# EUROPEAN PATENT SPECIFICATION

(11) **EP 1 266 641 B1**
(45) Date of publication and mention of the grant of the patent: **06.10.2004**
(21) Application number: 01830378.4
(22) Date of filing: 11.06.2001
(51) Int. Cl.: A61F 2/24

(54) **An annuloplasty prosthesis and a method for its manufacture**
Annuloplastieprothese und Herstellungsverfahren dafür
Prothèse pour annuloplastie et méthode pour sa manufacture

(43) Date of publication of application: 18.12.2002
(73) Proprietor: SORIN BIOMEDICA CARDIO S.p.A., 13040 Saluggia (Vercelli) (IT)
(72) Inventor: Arru, Pietro, 10020 Marentino (Torino) (IT); Bonetti, Francesco, 00161 Roma (IT); Stacchino, Carla, 10132 Torino (IT)
(74) Representative: Comoglio, Elena

(56) References cited:
- EP-A- 0 338 994

## Description

The present invention relates in general to a device for heart-valve repair operations and, in particular, to an annuloplasty prosthesis.

The human heart has four heart valves: the mitral valve, the tricuspid valve, the pulmonary valve, and the aortic valve.

The mitral valve is situated in the left atrio-ventricular ostium and controls the unidirectionality of the blood-flow from the atrium to the ventricle. It opens during the diastole and closes during the systole, preventing the blood from flowing back from the ventricle to the atrium. Disease or genetic defects may lead to deformation or dilation of the *annulus* of the mitral valve, causing it to operate incorrectly, with consequent back-flow of blood.

The same phenomenon may occur in the tricuspid valve which is situated between the right atrium and the right ventricle.

A method which is used to eliminate the back-flow phenomenon is that of re-establishing the correct shape and size of the valve *annulus* by surgical procedures known by the name of annuloplasty.

Annuloplasty consists of the surgical implantation of a support prosthesis on the dilated or deformed *annulus* in order to re-establish its physiological size and/or shape to enable the heart valve to operate correctly.

The support prostheses used in valve-repair operations take the name of annuloplasty prostheses. In most cases, a prosthesis of this type is constituted by a closed or open ring structure comprising an inner core and an outer covering of biocompatible material which enables the prosthesis to be sutured surgically.

Annuloplasty prostheses of various types of have been described in the prior art.

Initially, the prostheses proposed were predominantly of the rigid type in order drastically to reduce the dilation of the valve *annulus.* A prosthesis of this type is generally constituted by a metal core (for example, of titanium alloy or Elgiloy), an optional sheath covering the core, and an outer covering of fabric for suturing. Rigid annuloplasty prostheses are described, for example, in United States patents US 4 055 861, by Carpentier et al. published on 1 November 1977 and US 3 656 185 by Carpentier et al., published on 18th April 1972.

On the basis of the consideration that rigid prostheses interfere with the natural flexural movement of the *annulus* during the cardiac cycle, semi-rigid or fully flexible models have subsequently been proposed.

Semi-rigid annuloplasty prostheses are described, for example, in United States patents US 5 061 277 by Carpentier et al., published on 29th October 1991, US 5 104 407 by Lam et al., published on 14th April 1992, US 5 674 279 by Wright et al., published on 7th October 1997, US 5 824 066 by Gross et al., published on 20th October 1998, US 5 607 471 by Seguin et al., published on 4th March 1997, and US 6 143 024 by Campbell et al., published on 7th November 2000.

Fully flexible annuloplasty prostheses are described, for example, in United States patents US 5 041 130 by Carpentier et al., published on 20th August 1991, US 5 716 397 by Myers et al., published on 10th February 1998, US 6 102 945 by Campbell et al., published on 15th August 2000, and US 5 064 431 by Gilbertson et al., published on 12th November 1991.

Although rigid prostheses are satisfactory for some applications, they do not allow the *annulus* of the valve to bend along the base of the posterior cusp, with the result that they impose significant stresses on the suture points which are subjected to torsion and tension, and they prevent natural behaviour of the valve.

A fully flexible prostheses follows the movements of the *annulus* in an optimal manner during the cardiac cycle but does not enable its shape to be reconstructed in an optimal manner.

Moreover, since the above-mentioned known annuloplasty prostheses have structures in which the inner core and the outer covering are separate elements, they may cause the surgeon considerable difficulties in positioning the prosthesis and sewing it to the *annulus,* both due to relative sliding movements of the core and of the covering, and due to the fact that the prosthesis as a whole has a non-uniform consistency which may translate into difficulties of penetration of the needle into the prosthesis.

The document EP 0 338 994 A discloses a prosthetic annuloplasty device according to the preamble of claim 1.

A first object of the present invention is to provide an annuloplasty prosthesis which enables the dimensions and/or the physiological shape of the *annulus* to be re-established without, however, interfering with the natural flexural movement of the *annulus* during the cardiac cycle.

Another object of the present invention is to provide an annuloplasty prosthesis which has improved suturability, and which can be positioned and fixed more easily in the valve site of interest by sewing.

These objects are achieved by means of an annuloplasty prosthesis comprising at least one inner support element of elastomeric material and an outer fabric covering the support element, the elastomeric material impregnating the facing portions of the covering fabric so that the support element is fixed firmly to the covering fabric, preventing relative sliding movements between the fabric and the support element.

By virtue of this concept, the annuloplasty prosthesis of the present invention has a structure in which the covering fabric is fixed absolutely firmly to the support element, preventing relative movements between the support element and the covering, and facilitating the correct positioning of the prosthesis. This also permits the production of a flexible prosthesis having a particular shape which, for example, may be generally linear or circular, or may reproduce the natural shape of the *annulus* of a heart valve, for example, a mitral or tricuspid valve.

Moreover, the annuloplasty prosthesis of the present invention has greater tear resistance and a uniform soft consistency such as to ensure improved suturability of the prosthesis in the valve site of interest.

When increased stiffness properties are required, the prosthesis may also comprise a reinforcing element of material stiffer than the elastomeric material of the support element.

A further subject of the invention is a method of manufacturing an annuloplasty prosthesis as described above, comprising the steps of:
(a) providing a piece of covering fabric (20) on a support (10),
(b) impregnating at least a portion (40) of the outer face of the piece of fabric (20) with elastomeric material so as to produce at least one support element (50) of elastomeric material fixed firmly to the outer face of the fabric (20),
(c) removing the piece of fabric (20) from the support (10),
(d) rolling the piece of fabric (20) around its portion which is fixed firmly to the at least one support element (50) so as to produce a prosthesis (70) having at least one inner support element (50) and an outer fabric (20) in the form of a covering for the at least one support element (50),
(e) sewing the outermost free edge (22) of the covering fabric (20) along the edge of the prosthesis (70) with suture thread.

Yet a further subject of the invention is an annuloplasty kit comprising an annuloplasty prosthesis as described above, completely or partially coated with haemocompatible carbon, preferably turbostratic carbon, and a suture thread, also coated with the said carbon, for use for suturing the prosthesis to the valve *annulus*.

Further characteristics and advantages of the invention will become clear from the following detailed description, given purely by way of non-limiting example, with reference to the appended drawings, in which:
Figures 1 to 3 show, schematically, successive steps of the method for the manufacture of an annuloplasty prosthesis according to the invention,
Figure 4 is a view showing an annuloplasty prosthesis according to the invention, in section,
Figure 5 is a view showing a further embodiment of the annuloplasty prosthesis according to the invention, in section,
Figure 6 shows an intermediate stage of the method for the manufacture of a further embodiment of the annuloplasty prosthesis according to the invention,
Figure 7 is a view showing, in section, an annuloplasty prosthesis of the type shown in Figure 6, at an intermediate stage of the method of manufacture, and
Figure 8 shows an intermediate stage of the method of manufacturing yet another embodiment of the annuloplasty prosthesis according to the invention.

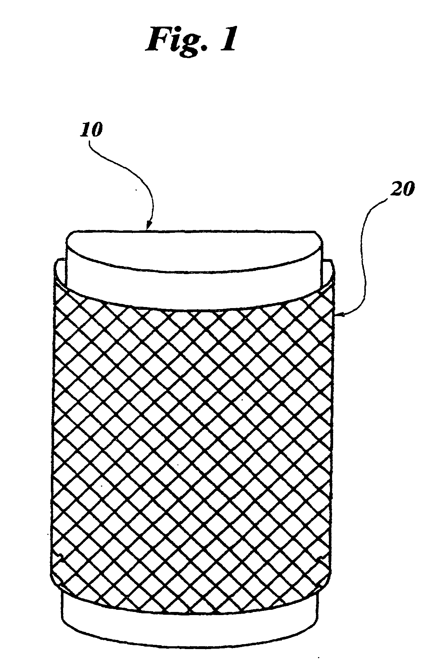

A support, which is used, in the manner of a mandrel, in a method for the manufacture of an annuloplasty prosthesis of a substantially circular shape or of a shape generally reproducing the geometry of a heart-valve *annulus,* is indicated 10 in Figure 1. A piece of fabric 20, in the form of a sleeve, is fitted on the support 10.

The fabric 20 is preferably made of a material selected from the group which consists of polyethylene terephthalate (PET), polytetrafluoroethylene (PTFE), polyethylene, and combinations thereof.

When the fabric sleeve 20 fitted on the support 10 is made of polyethylene terephthalate, it may advantageously be subjected to a heat treatment known as thermofixing which can produce a fabric of more homogeneous and stiffer texture such that the fabric 20 adopts the shape of the support 10 and retains it even after removal from the support.

This thermofixing treatment may be performed, for example, by putting the fabric sleeve 20, fitted on the support 10, in an oven at a temperature of least 100°C for a period of at least 20 minutes.

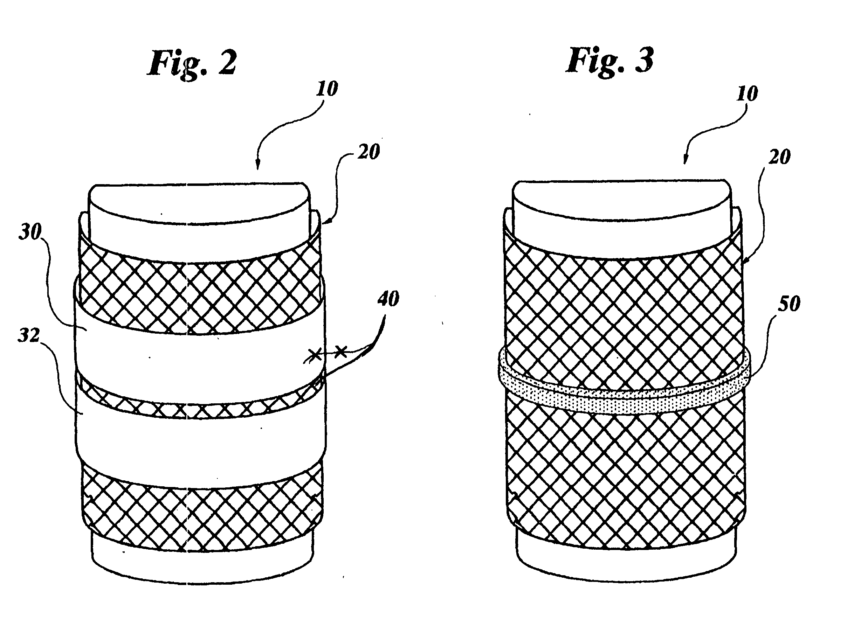

Figure 2 shows the fabric sleeve 20 fitted on the support 10, on which in turn are fitted two guide rings, 30 and 32, respectively, which cover underlying portions of the sleeve.

The guide rings 30 and 32 are spaced apart by a distance such that a strip 40 of fabric is exposed between them.

The outer face of the strip 40 is impregnated with elastomeric material.

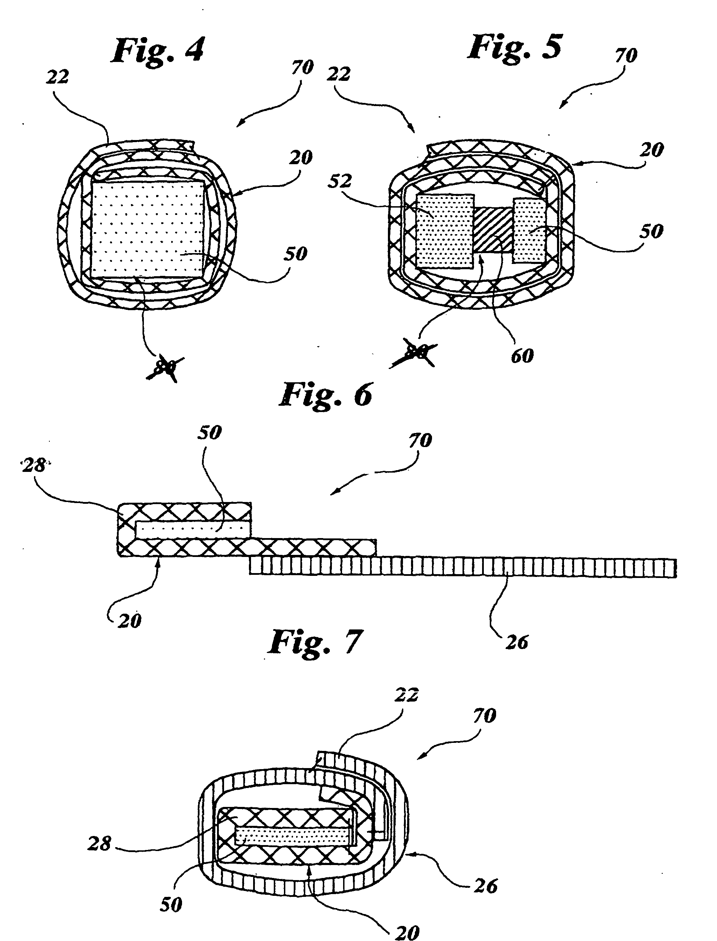

After the elastomeric material has been completely polymerized, the guide rings 30 and 32 are removed so that a support element 50 of elastomeric material is produced, fixed firmly to the outer face of the fabric sleeve 20, as shown in Figure 3.

The elastomeric material used for the support element 50 is preferably a material selected form the group which consists of silicone, polyurethane, and mixtures thereof.

Barium sulphate may also be added to the elastomeric material to render the prosthesis radiopaque.

Since the support element 50 is fixed absolutely firmly to the covering fabric 20, it is possible to produce a prosthesis in which relative sliding movements between the element and the fabric 20 are prevented.

This characteristic is particularly advantageous particularly in terms of improved suturability of the prosthesis and/or of its easier positioning in the valve site of interest.

Moreover, the presence of the impregnated elastomeric material in the fabric 20 gives the prosthesis a consistency such as to confer on it a particular shape which, for example, may be substantially circular or may reproduce the natural shape of a heart-valve *annulus*.

The fabric sleeve 20 is then slipped off the support 10, possibly cut to size, and rolled up around its portions which are fixed to the support element 50 so as to produce a prosthesis 70 having the fabric 20 as an outer covering for the inner support element 50 (Figure 4).

The outermost free edge 22 of the covering fabric 20 is sewn along the edge of the prosthesis 70 with suture thread.

The end edge portion of the outermost free edge 22 may be turned towards the inside in conventional manner, to produce a hem.

The prosthesis 70 thus produced may then be thermofixed in order to shrink the yarn of the fabric.

The support 10 shown in Figures 1 to 3 has a generally "D"-shaped cross-section which reproduces the geometry of a mitral-valve *annulus*.

The support 10 can therefore be used for the production of an annuloplasty prosthesis suitable for implantation in the site of a mitral valve.

According to further embodiments not specifically illustrated, however, the support 10 may be of a substantially circular shape or of a shape generally reproducing the geometry of the *annulus* of a tricuspid valve, or of other heart valves of interest.

Annuloplasty prostheses of the type described up to now, that is, comprising a single support element 50, are flexible prostheses.

However, the invention also comprises semi-rigid prosthesis as shown in Figure 5.

A prosthesis of this type comprises at least two support elements 50 and 52 and a reinforcing element 60 of material stiffer than the elastomeric material of the support elements 50, 52; this material is preferably selected from the group which consists of acetal polymers (such as, for example, polyoxymethylene), polypropylene, metal alloys such as, for example Co and Cr alloys, shape memory metals such as, for example, Nitinol, and combinations thereof.

Moreover, the number of support elements 50, 52 in the semi-rigid prosthesis of the invention may be selected substantially at will, in dependence on the requirements of use and on the required stiffness characteristics. The latter also determine the presence and number of reinforcing elements 60.

Moreover, according to an alternative embodiment of a semi-rigid prosthesis not shown specifically, the reinforcing element 60 is not interposed between two support elements 50 and 52 but is incorporated in the elastomeric material of the element 50.

In the embodiments described up to now, the annuloplasty prosthesis has a closed-ring structure.

However, the present invention also comprises embodiments not shown specifically in which the prosthesis is a ring structure which is open along one or more generatrices.

Annuloplasty prostheses according to these embodiments may be produced by a method exactly the same as that described above with reference to closed rings, in which the closed ring structure produced is cut along one or more generatrices.

The present invention also includes embodiments of the prosthesis in which the outer surface of the outer covering fabric 20 (see Figures 4 and 5) is completely or partially coated with a thin film of haemocompatible carbon, for example, turbostratic carbon. The method for the production of the turbostratic carbon film is described, for example, in the Applicant's patents US 5084151, US 5387247, US 5370684, US 5133845, and US 5423886.

The carbon coating may be formed on the piece of fabric 20 which is subsequently rolled up to form the prosthesis 70, or directly on the finished product. Moreover, the carbon coating may be formed on the entire surface of the covering fabric 20 or may be formed selectively purely on the portion of this surface which will come into contact with the blood.

The coating of the prosthesis with a thin layer of haemocompatible carbon, together with the selection of the material constituting the outer covering, contributes to improved haemocompatibility of the prosthesis and to controlled tissue growth by the receiving organism.

A prostheses completely or partially coated with haemocompatible carbon, preferably turbostratic carbon, may advantageously be combined with a suture thread, also coated with the haemocompatible carbon, to produce an annuloplasty kit, the elements of which are characterized by a high degree of haemocompatibility.

An alternative embodiment to that described above, in which the prosthesis is completely or partially coated with haemocompatible carbon is shown in Figures 6 and 7. In this embodiment, a pericardium strip 26 of animal origin which may subsequently be subjected to a detoxification process, for example, as described in the Applicant's US patent 5873812, is associated with the fabric sleeve 20 in the manner of an extension.

Figure 6 shows an intermediate stage of the method for the production of the annuloplasty prosthesis 70 according to this embodiment, in which a support element 50 has already been caused to adhere to the covering fabric 20 associated with the pericardium 26 of animal origin, by a method similar to that described above.

In the configuration of Figure 6, an edge portion 28 of the fabric 20 has already been wound around the support element 50, whereas the pericardium 26 is still in the extended condition.

The pericardium strip 26 and the remaining portion of tissue 20 are then rolled completely around the support element 50 to adopt the configuration shown in Figure 7, in which the pericardium 26 completely surrounds the ring.

According to this embodiment, the outermost portion of the covering is therefore made of pericardium of animal origin.

This embodiment has the advantage of increasing resistance to infective conditions such as endocarditis.

In the embodiments described up to now, the annuloplasty prosthesis is of a substantially circular shape or of a shape generally reproducing the geometry of a heart-valve *annulus*.

However, other embodiments in which the prosthesis has a substantially linear structure also fall within the scope of the invention.

An annuloplasty prosthesis in accordance with these embodiments may be produced from a flat piece of fabric by means of a method similar to that described above with reference to closed rings.

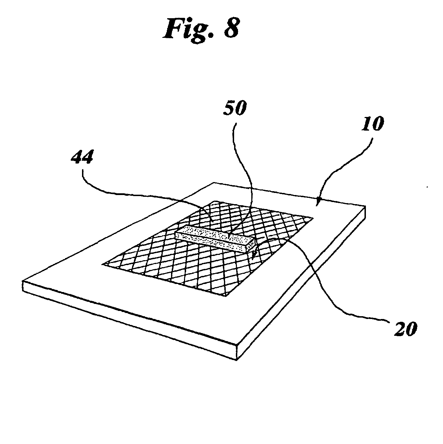

Figure 8 shows an intermediate stage of this method which corresponds to that shown in Figure 3 with reference to closed loops.

In Figure 8, the outer face of a flat piece of fabric 20 disposed on a flat support 10 has already been impregnated with elastomeric material so as to produce a support element 50 fixed firmly to the outer face of the flat piece 20.

The subsequent steps of the method, which are not shown specifically, are exactly the same as those described above with reference to closed rings. The flat piece of fabric 20 is removed from the support 10, possibly cut to size, and rolled around its portion which is fixed to the support element 50 so as to produce a linear structure having the covering fabric 20 as an outer covering of the inner support element 50.

Naturally, the principle of the invention remaining the same, the forms of embodiment and details of construction may be varied widely with respect to those described and illustrated purely by way of non-limiting example, without thereby departing from the scope of the invention as defined in the appended claims.

## Claims

1. An annuloplasty prosthesis (70) having at least one inner support element (50) and an outer fabric (20) covering the support element (50), **characterised in that** the support element (50) is made of elastomeric material, the elastomeric material impregnating the facing portions (40) of the covering fabric (20) so that the support element (50) is fixed firmly to the covering fabric (20), preventing relative sliding movements between the fabric (20) and the support element (50).

2. An annuloplasty prosthesis according to Claim 1 comprising at least one support element (50) of elastomeric material and at least one reinforcing element (60).

3. An annuloplasty prosthesis according to Claim 2 comprising two support elements (50, 52) between which a reinforcing element (60) is interposed.

4. An annuloplasty prosthesis according to Claim 2 in which the reinforcing element (60) is incorporated in the elastomeric material of the support element (50).

5. An annuloplasty prosthesis according to any one of Claims 1 to 4 in which the elastomeric material is selected from the group which consists of silicone, polyurethane, and mixtures thereof.

6. An annuloplasty prosthesis according to any one of Claims 1 to 5 in which the covering fabric (20) is made of a material selected from the group which consists of polyethylene terephthalate, polytetrafluoroethylene, polyethylene, and combinations thereof.

7. An annuloplasty prosthesis according to any one of Claims 1 to 6, of substantially circular shape.

8. An annuloplasty prosthesis according to any one of Claims 1 to 6, of a shape generally reproducing the geometry of a heart-valve *annulus*.

9. An annuloplasty prosthesis according to Claim 8 of a shape generally reproducing the geometry of a mitral-valve *annulus.*

10. An annuloplasty prosthesis according to Claim 8, of a shape generally reproducing the geometry of a tricuspid-valve *annulus*.

11. An annuloplasty prosthesis according to any one of Claims 7 to 10, which is a closed ring.

12. An annuloplasty prosthesis according to any one of Claims 7 to 10 which is a ring which is open along one or more generatrices.

13. An annuloplasty prosthesis according to any one of Claims 1 to 6, of substantially linear shape.

14. An annuloplasty prosthesis according to any one of Claims 1 to 13 in which at least a portion of the surface of the prosthesis (70) is coated with haemocompatible carbon.

15. An annuloplasty prosthesis according to Claim 14 in which the haemocompatible carbon is turbostratic carbon.

16. An annuloplasty prosthesis according to any one of Claims 1 to 13 in which pericardium of animal origin (26) is associated with the covering fabric (20) so as to be wrapped around it.

17. An annuloplasty kit comprising an annuloplasty prosthesis according to Claim 14 and suture thread coated with haemocompatible carbon for suturing the prosthesis to the heart muscle.

18. An annuloplasty kit according to Claim 17 in which the haemocompatible carbon is turbostratic carbon.

19. A method of manufacturing an annuloplasty prosthesis according to any one of the preceding claims, comprising the steps of:
(a) providing a piece of covering fabric (20) on a support (10),
(b) impregnating at least a portion (40) of the outer face of the piece of fabric (20) with elastomeric material so as to produce at least one support element (50) of elastomeric material fixed firmly to the outer face of the fabric (20),
(c) removing the piece of fabric (20) from the support (10),
(d) rolling the piece of fabric (20) around its portion which is fixed firmly to the at least one support element (50) so as to produce a prosthesis (70) having at least one inner support element (50) and an outer fabric (20) in the form of a covering for the at least one support element (50),
(e) sewing the outermost free edge (22) of the covering fabric (20) along the edge of the prosthesis (70) with suture thread.

20. A method according to Claim 19 in which, with reference to step a), the piece of fabric (20) is in the form of a sleeve fitted on the support (10), which has a substantially circular cross-section or a cross-section generally reproducing the geometry of a heart-valve *annulus*.

21. A method according to Claim 20 in which the covering fabric (20) is made of polyethylene terephthalate, the method further comprising, after the step (a) of fitting the fabric sleeve (20) on the support (10), a step (a₂) of subjecting the fabric (20) to a heat treatment such that the fabric (20) adopts the shape of the support (10) and retains it after removal from the support.

22. A method according to Claim 19 in which, with reference to step a), the piece of fabric (20) is in the form of a flat piece placed on the support (10), which is substantially flat.

## Patentansprüche

1. Annuloplastik-Prothese (70), die zumindest ein inneres Stützelement (50) sowie ein äußeres Gewebe (20) besitzt, das das Stützelement (50) bedeckt, **dadurch gekennzeichnet, dass** das Stützelement (50) aus einem elastomeren Material hergestellt ist, wobei das elastomere Material die gegenüberliegenden Teile (40) des abdeckenden Gewebes (20) imprägniert, so dass das Stützelement (50) fest am abdeckenden Gewebe (20) befestigt ist, um relative Gleitbewegungen zwischen dem Gewebe (20) und dem Stützelement (50) zu verhindern.

2. Annuloplastik-Prothese gemäß Anspruch 1, wobei die Prothese zumindest ein Stützelement (50) aus einem elastomeren Material sowie zumindest ein Verstärkungselement (60) enthält.

3. Annuloplastik-Prothese gemäß Anspruch 2, wobei die Prothese zwei Stützelemente (50, 52) enthält, zwischen die ein Verstärkungselement (60) eingesetzt ist.

4. Annuloplastik-Prothese gemäß Anspruch 2, wobei das Verstärkungselement (60) im elastomeren Material des Stützelements (50) enthalten ist.

5. Annuloplastik-Prothese gemäß irgendeinem der Ansprüche 1 bis 4, wobei das elastomere Material aus einer Gruppe ausgewählt wird, die Silikon, Polyurethan und Gemische davon enthält.

6. Annuloplastik-Prothese gemäß irgendeinem der Ansprüche 1 bis 5, wobei das abdeckende Gewebe (20) aus einem Material hergestellt wird, das aus einer Gruppe ausgewählt wird, die aus Polyäthylenterephtalat, Polytetrafluoräthylen, Polyäthylen sowie Kombinationen davon besteht.

7. Annuloplastik-Prothese gemäß irgendeinem der Ansprüche 1 bis 6, wobei die Prothese im Wesentlichen kreisförmig ausgebildet ist.

8. Annuloplastik-Prothese gemäß irgendeinem der Ansprüche 1 bis 6, wobei die Prothese eine Form besitzt, die im Allgemeinen die Geometrie eines Herzklappen-Annulus wiedergibt.

9. Annuloplastik-Prothese gemäß Anspruch 8, wobei die Prothese eine Form besitzt, die im Allgemeinen die Geometrie eines Mitralklappen-Annulus wiedergibt.

10. Annuloplastik-Prothese gemäß Anspruch 8, wobei die Prothese eine Form besitzt, die im Allgemeinen die Geometrie eines Trikuspidalklappen-Annulus wiedergibt.

11. Annuloplastik-Prothese gemäß irgendeinem der Ansprüche 7 bis 10, wobei die Prothese ein geschlossener Ring ist.

12. Annuloplastik-Prothese gemäß irgendeinem der Ansprüche 7 bis 10, wobei die Prothese ein Ring ist, der längs einer oder mehrerer Erzeugenden geöffnet ist.

13. Annuloplastik-Prothese gemäß irgendeinem der Ansprüche 1 bis 6, wobei die Prothese im Wesentlichen eine lineare Form besitzt.

14. Annuloplastik-Prothese gemäß irgendeinem der Ansprüche 1 bis 13, wobei zumindest ein Teil der Oberfläche der Prothese (70) mit hämokompatiblem Kohlenstoff beschichtet ist.

15. Annuloplastik-Prothese gemäß Anspruch 14, wobei der hämokompatible Kohlenstoff turbostratischer Kohlenstoff ist.

16. Annuloplastik-Prothese gemäß irgendeinem der Ansprüche 1 bis 13, wobei Perikard tierischen Ursprungs (26) dem abdeckenden Gewebe (20) zugeordnet ist, um es um dieses zu schlingen.

17. Annuloplastik-Set, wobei das Set eine Annuloplastik-Prothese gemäß Anspruch 14 sowie ein Nahtmaterial enthält, das mit hämokompatiblem Kohlenstoff beschichtet ist, um die Prothese am Herzmuskel anzunähen.

18. Annuloplastik-Set gemäß Anspruch 17, wobei der hämokompatible Kohlenstoff turbostatischer Kohlenstoff ist.

19. Verfahren zur Herstellung einer Annuloplastik-Prothese gemäß irgendeinem der bisherigen Ansprüche, wobei das Verfahren folgende Schritte enthält
(a) Bereitstellen eines Stücks eines abdeckenden Gewebes (20) auf einer Halterung (10),
(b) Imprägnieren von zumindest einem Teil (40) der Außenfläche des Stücks des Gewebes (20) mit einem elastomeren Material, um zumindest ein Stützelement (50) des elastomeren Materials herzustellen, das an der Außenfläche des Gewebes (20) fest abgebracht wird,
(c) Entfernen des Stücks des Gewebes (20) von der Halterung (10),
(d) Rollen des Stücks des Gewebes (20) rund um seinen Teil, der an dem zumindest einen Stützelement (50) fest angebracht ist, um eine Prothese (70) herzustellen, die zumindest ein inneres Stützelement (50) sowie ein äußeres Gewebe (20) in Form einer Abdeckung für das zumindest eine Stützelement (50) besitzt,
(e) Annähen des äußersten freien Rands (22) des abdeckenden Gewebes (20) entlang des Rands der Prothese (70) mit einem Nahtmaterial.

20. Verfahren gemäß Anspruch 19, wobei im Hinblick auf Schritt a) das Stück des Gewebes (20) die Form einer Hülse besitzt, die auf die Halterung (10) gesetzt wird, die einen im Wesentlichen kreisförmigen Querschnitt oder einen Querschnitt besitzt, der im Allgemeinen die Geometrie eines Herzklappen-Annulus wiedergibt.

21. Verfahren gemäß Anspruch 20, wobei das abdeckende Gewebe (20) aus Polyäthylenterephtalat hergestellt ist, wobei das Verfahren weiters nach dem Schritt (a), in dem die Gewebehülse (20) auf die Halterung (10) aufgesetzt wird, einen Schritt (a₂) besitzt, in dem das Gewebe (20) einer Wärmebehandlung ausgesetzt wird, so dass das Gewebe (20) die Form der Halterung (10) annimmt und diese nach dem Entfernen von der Halterung beibehält.

22. Verfahren gemäß Anspruch 19, wobei im Hinblick auf den Schritt a) das Stück des Gewebes (20) die Form eines flachen Stücks besitzt, das auf der Halterung (10) angeordnet wird, die im Wesentlichen flach ist.

## Revendications

1. Prothèse pour annuloplastie (70) ayant au moins un élément de support intérieur (50) et un tissu extérieur (20) couvrant l'élément de support (50), **caractérisée en ce que** l'élément de support (50) est fabriqué dans un matériau élastomère, le matériau élastomère imprégnant les portions de revers (40) du tissu de couverture (20) de sorte que l'élément de support (50) est fixé fermement sur le tissu de couverture (20), empêchant des mouvements de glissage relatif entre le tissu (20) et l'élément de support (50).

2. Prothèse pour annyloplastie selon la revendication 1, comprenant au moins un élément de support (50) de matériau élastomère et au moins un élément de renfort (60).

3. Prothèse pour annuloplastie selon la revendication 2, comprenant deux éléments de support (50, 52) entre lesquels un élément de renfort (60) est interposé.

4. Prothèse pour annuloplastie selon la revendication 2, dans laquelle l'élément de renfort (60) est incorporé dans le matériau élastomère de l'élément de support (50).

5. Prothèse pour annuloplastie selon l'une quelconque des revendications 1 à 4, dans laquelle le matériau élastomère est choisi à partir du groupe composé de la silicone, du polyuréthane, et de mélanges de ceux-ci.

6. Prothèse pour annuloplastie selon l'une quelconque des revendications 1 à 5, dans laquelle le tissu de couverture (20) est fabriqué dans un matériau choisi dans le groupe composé du polyéthylène téréphtalate, du polytétrafluoréthylène, du polyéthylène, et de combinaisons de ceux-ci.

7. Prothèse pour annuloplastie selon l'une quelconque des revendications 1 à 6, de forme sensiblement circulaire.

8. Prothèse pour annuloplastie selon l'une quelconque des revendications 1 à 6, d'une forme reproduisant généralement la géométrie d'un anneau de valve cardiaque.

9. Prothèse pour annuloplastie selon la revendication 8, d'une forme reproduisant généralement la géométrie d'un anneau de valve mitrale.

10. Prothèse pour annuloplastie selon la revendication 8, d'une forme reproduisant généralement la géométrie d'un anneau de valve tricuspide.

11. Prothèse pour annuloplastie selon l'une quelconque des revendications 7 à 10, qui est une bague fermée.

12. Prothèse pour annuloplastie selon l'une quelconque des revendications 7 à 10, qui est une bague qui est ouverte le long d'une ou plus génératrices.

13. Prothèse pour annuloplastie selon l'une quelconque des revendications 1 à 6, de forme sensiblement linéaire.

14. Prothèse pour annuloplastie selon l'une quelconque des revendications 1 à 13, dans laquelle au moins une portion de la surface de la prothèse (70) est recouverte de carbone hémocompatible.

15. Prothèse pour annuloplastie selon la revendication 14, dans laquelle le carbone hémocompatible est du carbone turbostratique.

16. Prothèse pour annuloplastie selon l'une quelconque des revendications 1 à 13, dans laquelle le péricarde d'origine animale (26) est associé avec le tissu de couverture (20) de manière à être enveloppé autour.

17. Kit pour annuloplastie comprenant une prothèse pour annuloplastie selon la revendication 14 et du fil pour suture recouvert de carbone hémocompatible pour suturer la prothèse au muscle cardiaque.

18. Kit pour annuloplastie selon la revendication 17, dans lequel le carbone hémocompatible est du carbone turbostratique.

19. Procédé de fabrication d'une prothèse pour annuloplastie selon l'une quelconque des revendications précédentes, comprenant les étapes consistant à :
(a) fournir un morceau de tissu de couverture (20) sur un support (10),
(b) imprégner au moins une portion (40) de la face extérieure du morceau de tissu (20) avec du matériau élastomère de manière à produire au moins un élément de support (50) de matériau élastomère fixé fermement sur la surface extérieure du tissu (20),
(c) retirer le morceau de tissu (20) du support (10),
(d) enrouler le morceau de tissu (20) autour de sa portion qui est fixée fermement sur le au moins un élément de support (50) de manière à produire une prothèse (70) ayant au moins un élément de support intérieur (50) et un tissu extérieur (20) sous la forme d'un revêtement pour le au moins un élément de support (50),
(e) coudre le bord libre le plus extérieur (22) du tissu de couverture (20) le long du bord de la prothèse (70) avec du fil de suture.

20. Procédé selon la revendication 19, dans lequel, en référence à l'étape (a), le morceau de tissu (20) a la forme d'un manchon ajusté sur le support (10), qui a une section sensiblement circulaire ou une section reproduisant généralement la géométrie d'un anneau de valve cardiaque.

21. Procédé selon la revendication 20, dans lequel le tissu de couverture (20) est fabriqué en polyéthylène téréphtalate, le procédé comprenant en outre, après l'étage
(a) consistant à ajuster le manchon de tissu (20) sur le support (10), une étape (a₂) consistant à soumettre le tissu (20) à un traitement thermique de manière à ce que le tissu (20) adopte la forme du support (10) et la conserve après le retrait du support.

22. Procédé selon la revendication 19, dans lequel, en référence à l'étape (a), le morceau de tissu (20) a la forme d'un morceau plat placé sur le support (10), qui est sensiblement plat.
